# EUROPÄISCHE PATENTSCHRIFT

(11) **EP 3 858 156 B1**
(45) Veröffentlichungstag und Bekanntmachung des Hinweises auf die Patenterteilung: **11.10.2023**
(21) Anmeldenummer: 21154348.3
(22) Anmeldetag: 29.01.2021
(51) Int. Cl.: A23L 33/105, A23L 33/135, A23L 33/155, A23L 33/16, A23L 33/175, A61K 31/122, A61K 31/198, A61K 31/375, A61K 31/4188, A61K 31/4415, A61K 31/455, A61K 31/495, A61K 31/51, A61K 31/519, A61K 31/592, A61K 31/714, A61K 33/04, A61K 33/34, A61K 35/745, A61K 35/747

(54) **2-PHASEN-PRÄPARAT FÜR REISEN**
2-PHASE PREPARATION FOR TRAVEL
PRÉPARATION BIPHASIQUE POUR VOYAGES

(30) Priorität: 31.01.2020 DE 202020100539 U
(43) Veröffentlichungstag der Anmeldung: 04.08.2021
(73) Patentinhaber: Humanicon GmbH, 20251 Hamburg (DE)
(72) Erfinder: Bamberger, Christoph Marcus, 20149 Hamburg (DE)
(74) Vertreter: Stork Bamberger Patentanwälte PartmbB

(56) Entgegenhaltungen:
- EP-A1- 2 737 809
- EP-B1- 2 852 438
- DE-U1-202018 005 641
- US-A1- 2009 104 171
- DATABASE GNPD [Online] MINTEL; 20. Januar 2005 (2005-01-20), anonymous: "Tropical Flavor Dietary Supplement", XP55813554, Database accession no. 10204258
- DATABASE GNPD [Online] MINTEL; 2. Dezember 2019 (2019-12-02), anonymous: "Hair Vitamins Food Supplement", XP055813558, Database accession no. 7076939
- DATABASE GNPD [Online] MINTEL; 26. Juni 2019 (2019-06-26), anonymous: "Premium Liver Care Dietary Supplement", XP55813560, Database accession no. 6640983
- Arendt Josephine: "Approaches to the Pharmacological Management of Jet Lag", Drugs, vol. 78, no. 14, 1 September 2018 (2018-09-01), pages 1419-1431, XP055811059, NZ ISSN: 0012-6667, DOI: 10.1007/s40265-018-0973-8 Retrieved from the Internet: URL:https://link.springer.com/content/pdf/ 10.1007/s40265-018-0973-8.pdf>

## Beschreibung

Unsere Gesellschaft ist in den vergangenen Jahren und Jahrzehnten zunehmend mobil geworden. Reisen, gerade auch in weit entfernte Länder, erfreuen sich großer Beliebtheit, und auch aus beruflichen Gründen reisen immer mehr Menschen zu weiter entfernten Zielen. Weil man mit dem Flugzeug reisen kann, sind auch Ziele in großer Entfernung innerhalb eines Tages erreichbar.

So angenehm es ist, Ziele auf der ganzen Welt innerhalb weniger Stunden erreichen zu können, so unangenehm sind oft die Auswirkungen auf den Körper, die mit solchen Reisen verbunden sind. Zu nennen ist insbesondere der sogenannte "Jet-Lag", also die Anpassungsschwierigkeiten des Körpers an eine neue Zeitzone, die auftreten, wenn man innerhalb kurzer Zeit mehrere Zeitzonen in westliche oder östliche Richtung passiert.

Aber auch unabhängig von der Anzahl der durchreisten Zeitzonen können Schwierigkeiten auftreten, die durch das Fortbewegungsmittel oder die gegenüber der gewohnten Umgebung und Lebensweise geänderten äußeren Bedingungen und Gegebenheiten hervorgerufen werden. So besteht aufgrund der trockenen Luft in Flugzeugen die Gefahr der Austrocknung von Haut und Haaren. Haut und Haare werden im Urlaub durch längeren Aufenthalt im Freien und insbesondere in der Sonne beansprucht. Die Gefahr der beschleunigten Hautalterung durch intensive Sonneneinwirkung ist nachgewiesen.

An fremden Orten ist der Körper ungewohnten Keimen ausgesetzt, die das Immunsystem in höherem Maße als zu Hause beanspruchen. Gerade auch der Magen- und Darmtrakt ist auf Reisen angesichts für den Körper ungewohnter Speisen und Getränke besonders anfällig. Zu all diesem gesellt sich oftmals eine ungesündere Lebensführung als im Alltag, beispielsweise aufgrund von erhöhtem Alkoholkonsum oder dem Essen von weniger gesunden Lebensmitteln.

Wünschenswert wäre es, wenn man all diesen Beschwerden auf einfache Weise vorbeugen oder sie bei ihrem Auftreten auf einfache Weise lindern könnte. Zwar sind Präparate bekannt, die einzelne Probleme adressieren. So ist z. B. in der DE 10 2014 017 455 A1 ein Kreatin enthaltendes Nahrungsergänzungsmittel beschrieben, das den Schlaf verbessern und eine Anpassung an neue Zeitzonen beschleunigen soll. Ein anderes Präparat zur Linderung von Schlafstörungen, auch hervorgerufen durch Jet-Lag ist in der US 2013/064804 A1 offenbart. Die WO 2006/089317 A1 betrifft eine Wirkstoffmischung enthaltend ein Flavonoid zur Einnahme vor längeren Reisen zur Thromboseprophylaxe. DE 20 2018 005641 U1 beschreibt ein Nahrungsergänzungsmittel zur Vorbeugung oder Behandlung von Ein- und Durchschlafstörungen enthaltend L-Tryptophan und/oder Melatonin und Passionsblumenextrakt. EP2 852 438 offenbart eine Zusammensetzung zur Behandlung einer Störung des circadianen Rhythmus, das NADH und D-Galaktose sowie Vitamin C, Magnesiumcitrat, Tryptophan sowie weitere Spurenenlemente in einer Phase enthalten kann. Ein kommerzielles Produkt zur Verhinderung von Hangover und Jetlag enthält B-Vitamine (GNPD-Datenbank, Eintrag 10204258, 20-01-2005, www.gnpd.com). Es ist jedoch kein Präparat bekannt, das in allgemeiner Form die Hauptbeschwerden, die mit längeren Reisen verbunden sind, adressiert und diese entweder präventiv behandelt oder bei ihrem Auftreten lindert bzw. beseitigt. Aufgabe der vorliegenden Erfindung war es daher, ein Präparat zur Verfügung zu stellen, dass die vorstehend genannten Probleme und Beschwerden, die durch Reisen hervorgerufen werden, zu adressieren.

Diese Aufgabe wird durch ein zirkadianes Nahrungsergänzungsmittel zur Verwendung bei der Vorbeugung vor und Linderung von reisebedingten Beschwerden gelöst, das aus einem 2-Phasen-Präparat besteht, wobei die erste Phase D-Biotin (auch als Vitamin H bezeichnet) und Vitamin C enthält, die zweite Phase Magnesium und Tryptophan enthält und mindestens eine der beiden Phasen weiterhin Folgendes enthält: Cyanocobalamin, Coenzym Q10, Laktobakterien und Bifidobakterien, Zink, Vitamin D, Kupfer, Selen, Ingwer-Extrakt, Curcuma-Extrakt, Cranberry-Extrakt, Mariendistelextrakt sowie optional eine oder mehrere der folgenden Substanzen: L-Glutamin, Nicotinamid, Vitamin B6, Vitamin B1, Folsäure, Melissenextrakt und Passionsblumenextrakt, wobei die erste Phase morgens und die zweite Phase abends eingenommen wird.

Ein solches erfindungsgemäßes Präparat beugt reisebedingten Problemen vor. Es lindert Unwohlsein, das beim Reisen auftritt, und mildert Beschwerden, die bedingt durch gegenüber dem Zuhause veränderte Umstände, wie verändertes Klima und veränderter Tagesablauf, hervorgerufen werden. Das Präparat ist proenergetisch und probiotisch.

Besonders geeignet ist das erfindungsgemäße Präparat für Reisen über mehrere Zeitzonen und/oder in andere Klimazonen und/oder bei Flugreisen. D. h. das erfindungsgemäße Präparat beugt besonders gut solchen Beschwerden vor bzw. lindert solche Beschwerden, wie sie auf Reisen, die über mehrere Zeitzonen und/oder in anderen Klimazonen gehen und/oder Flugreisen sind, auftreten. Insbesondere bei Fernreisen treten üblicherweise zum einen bei einer größeren Anzahl von Menschen Beschwerden auf als bei kürzeren Reisen, außerdem sind die Beschwerden oft zahlreicher und verschiedenartiger als bei kürzeren Reisen. Bei Flugreisen sind auch auf kürzeren Strecken oftmals Reisebeschwerden zu beobachten, für die das erfindungsgemäße Präparat ebenfalls besonders geeignet ist.

Besonders bevorzugt enthält das erfindungsgemäße Präparat als Wirkstoffe nur die vorstehend genannten Substanzen.

Unter einem zirkadianen Nahrungsergänzungsmittel wird ein tagesrhythmisch angepasstes Nahrungsergänzungsmittel verstanden. Die beiden Phasen des Präparats werden zu unterschiedlichen Tageszeiten eingenommen, einmal morgens und einmal abends. Die erste (morgendliche) Phase wird nach dem Aufstehen, vorzugsweise nach dem Frühstück, eingenommen. Die zweite (abendliche) Phase wird abends vor dem Schlafengehen, vorzugsweise eine Stunde vor dem Schlafengehen, eingenommen.

D-Biotin und Vitamin C wirken proenergetisch und regen den Kreislauf an. Magnesium und Tryptophan wirken ebenso wie Kupfer und L-Glutamin beruhigend. Diese auf die beiden Phasen verteilte zirkadiane (tagesrhythmische) Dosierung der Wirkstoffe beeinflusst den Schlaf-Wach-Rhythmus positiv und beschleunigt eine Anpassung des Körpers an eine neue Zeitzone.

D-Biotin ist vorzugsweise in einer Menge von 0,01 mg bis 0,1 mg, insbesondere 0,02 mg bis 0,08 mg und besonders bevorzugt zu 0,05 mg, in dem Präparat enthalten.

Die angegebenen Wirkstoffmengen wie auch alle im Weiteren angegebenen Wirkstoffmengen beziehen sich immer auf die Gesamtwirkstoffmenge einer Einheit des Präparats, bestehend aus den beiden Phasen. Etwaige Gegenionen bei Salzen sind bei den Mengenangaben nicht berücksichtigt. Die Angaben beziehen sich auf die Wirkstoff-Einheit. Eine Einheit des Präparats entspricht vorzugsweise einer Tagesdosis. Sofern nichts anderes angegeben ist, können die Wirkstoffe sowohl ausschließlich in der ersten Phase wie auch ausschließlich in der zweiten Phase enthalten sein. Ebenso ist es möglich, die Verbindungen in beliebigem Verhältnis auf die beiden Phasen aufzuteilen.

Vitamin C ist vorzugsweise in einer Menge von 10 mg bis 200 mg, insbesondere 50 mg bis 100 mg und besonders bevorzugt 60 mg bis 80 mg, in dem Präparat enthalten. Vorzugsweise ist das Vitamin C als Calciumascorbat oder Natriumascorbat enthalten, wobei Calciumascorbat besonders bevorzugt ist.

Cyanocobalamin ist eine biologisch inaktive Form aus der Gruppe der Cobalamine, die auch als Vitamin B12-Gruppe bekannt sind. Cyanocobalamin ist vorzugsweise in einer Menge von 0,0001 mg bis 0,1 mg, insbesondere 0,001 mg bis 0,008 mg und besonders bevorzugt 0,004 mg bis 0,006 mg, in dem Präparat enthalten.

Bevorzugte Mengen für Magnesium in dem Präparat liegen im Bereich von 10 mg bis 200 mg, insbesondere von 40 mg bis 100 mg und besonders bevorzugt von 50 mg bis 70 mg. Vorzugsweise ist das Magnesium als Magnesiumoxid, Magnesiumcitrat, Magnesiumhydrogencitrat, Trimagnesiumdicitrat oder Magnesiumcarbonat enthalten, wobei Magnesiumoxid, Magnesiumcitrat, Magnesiumhydrogencitrat, Trimagnesiumdicitrat besonders bevorzugt sind.

Bevorzugte Mengen für Tryptophan in dem Präparat liegen im Bereich von 10 mg bis 200 mg, insbesondere von 50 mg bis 150 mg und besonders bevorzugt von 80 mg bis 120 mg. Unter "Tryptophan" im Sinne dieses Patentes ist L-Tryptophan zu verstehen.

Die enthaltenen Laktobakterien und Bifidobakterien stärken die Darmflora und mildern damit Belastungen, die auf den Darm einwirken, ab. Zink schützt Haut und Haar, stärkt das Immunsystem und wirkt einem Austrocknen entgegen. Vitamin D verbessert ebenfalls die Immunabwehr und schützt so den Körper vor den Herausforderungen, die mit den für den Körper fremden Keimen der neuen Umgebung verbunden sind.

Zink ist vorzugsweise in einer Menge von 5 mg bis 20 mg, insbesondere 7 mg bis 12 mg und besonders bevorzugt zu 10 mg, in dem Präparat enthalten. Vorzugsweise ist das Zink als Zinkcitrat, Zinksulfat, Zinkoxid, Zinkgluconat oder Zinkacetat enthalten, wobei Zinkcitrat besonders bevorzugt ist.

Vitamin D bezeichnet in der vorliegenden Anmeldung Vitamin D3, das auch unter dem Namen Cholecalciferol bekannt ist. Vitamin D ist vorzugsweise in einer Menge von 0,002 mg bis 0,1 mg, insbesondere 0,01 mg bis 0,05 mg und besonders bevorzugt zu 0,02 mg, in dem Präparat enthalten.

Zink und Vitamin D können sowohl in der ersten Phase wie auch in der zweiten Phase enthalten sein. Ebenso ist es möglich, die Verbindungen in beliebigem Verhältnis auf die beiden Phasen aufzuteilen. Besonders bevorzugt sind die beiden Wirkstoffe in der ersten Phase enthalten.

Selen ist vorzugsweise in einer Menge von 0,01 mg bis 0,2 mg, insbesondere 0,05 mg bis 0,1 mg und besonders bevorzugt von 0,06 mg bis 0,07 mg, in dem Präparat enthalten. Vorzugsweise ist das Selen als Natriumselenit enthalten.

Kupfer ist vorzugsweise in einer Menge von 0,1 mg bis 1 mg, insbesondere 0,2 mg bis 0,8 mg und besonders bevorzugt zu 0,5 mg, in dem Präparat enthalten. Vorzugsweise ist das Kupfer als Kupfergluconat, Kupfercitrat oder Kupfersulfat enthalten, wobei Kupfergluconat besonders bevorzugt ist.

Als Curcuma-Extrakt wird vorzugsweise Curcuma longa-Extrakt eingesetzt. Dieser Extrakt hat einen hohen Gehalt an Curcumin als Wirkstoff; der Curcumin-Gehalt des Extrakts liegt üblicherweise zwischen 60 % und 80 %. Curcuma-Extrakt kommt vorzugsweise in einer Menge von 10 mg. bis 200 mg, insbesondere 30 mg bis 100 mg und insbesondere 60 bis 80 mg zum Einsatz. Bevorzugte Curcumin-Gehalte des Präparats liegen bei 6 mg bis 160 mg, insbesondere 10 mg bis 100 mg und besonders bevorzugt 30 mg bis 60 mg.

Ingwer (lat. Name: Zingiber officinale)-Extrakt enthält als Wirkstoff Gingerol. Vorzugsweise wird ein Ingwerwurzel-Extrakt eingesetzt. Ingwer-Extrakt kommt vorzugsweise in einer Menge von 10 mg. bis 200 mg, insbesondere 30 mg bis 100 mg und besonders bevorzugt 60 bis 80 mg zum Einsatz. Bevorzugte Gingerol-Gehalte des Präparats liegen bei 0,5 mg bis 10 mg, insbesondere 1 mg bis 7 mg und besonders bevorzugt 3 mg bis 4 mg.

Cranberry (lat. Name: Vaccinium macrocarpon)-Extrakt enthält als Wirkstoff Proanthocyanidine. Cranberry-Extrakt kommt vorzugsweise in einer Menge von 10 mg bis 200 mg, insbesondere 20 mg bis 100 mg und besonders bevorzugt 40 bis 60 mg zum Einsatz. Bevorzugte Proanthocyanidin-Gehalte des Präparats liegen bei 2 mg bis 40 mg, insbesondere 5 mg bis 20 mg und besonders bevorzugt 10 mg bis 15 mg.

Mariendistel (lat. Name: Silybum marianum)-Extrakt enthält als Wirkstoff Silymarin. Mariendistel-Extrakt kommt vorzugsweise in einer Menge von 10 mg bis 500 mg, insbesondere 50 mg bis 200 mg und besonders bevorzugt 80 bis 120 mg zum Einsatz. Bevorzugte Silymarin-Gehalte des Präparats liegen bei 20 mg bis 200 mg, insbesondere 40 mg bis 120 mg und besonders bevorzugt 60 mg bis 100 mg.

Vorzugsweise besteht das Nahrungsergänzungsmittel aus einem 2-Phasen-Präparat, wobei die erste Phase D-Biotin und Vitamin C enthält, die zweite Phase Magnesium und Tryptophan enthält und mindestens eine der beiden Phasen weiterhin Cyanocobalamin, Coenzym Q10, Laktobakterien und Bifidobakterien, Zink, Vitamin D, Kupfer, Selen, Ingwer-Extrakt, Curcuma-Extrakt, Cranberry-Extrakt und Mariendistelextrakt enthält, wobei besonders bevorzugt keine weiteren Wirkstoffe enthalten sind.

Besonders bevorzugt ist ein Präparat, bei dem mindestens eine der beiden Phasen neben den vorstehend genannten Wirkstoffen weiterhin mindestens eine der folgenden Wirkstoffe enthält:
L-Glutamin, Nicotinamid, Vitamin B6, Vitamin B1 und Folsäure.

Cyanocobalamin, Coenzym Q10, Laktobakterien und Bifidobakterien, Zink, Vitamin D, Kupfer, Selen, Ingwer-Extrakt, Curcuma-Extrakt, Cranberry-Extrakt, Mariendistelextrakt sowie optional eine oder mehrere der folgenden Substanzen: L-Glutamin, Nicotinamid, Vitamin B6, Vitamin B1, Folsäure können in einer der beiden Phasen oder auch in beiden enthalten sein. Besonders günstig ist es, wenn von den vorstehend genannten Verbindungen Coenzym Q10, Curcuma-Extrakt, Ingwer-Extrakt, Cranberry-Extrakt und Selen neben Biotin und Vitamin C in der ersten Phase enthalten sind.

Weiterhin ist es bevorzugt, wenn Mariendistelextrakt, Cyanocobalamin und Kupfer sowie, sofern L-Glutamin, Vitamin B6, Vitamin B1 und/oder Folsäure enthalten sind, diese neben Magnesium und Tryptophan in der zweiten Phase enthalten sind.

Besonders bevorzugt enthält das Präparat L-Glutamin. L-Glutamin ist vorzugsweise in einer Menge von 5 mg bis 50 mg, insbesondere 10 mg bis 40 mg und besonders bevorzugt zu 25 mg, in dem Präparat enthalten.

Besonders bevorzugt enthält das Präparat Vitamin B6. Vitamin B6 bezeichnet in der vorliegenden Anmeldung Pyridoxin. Vitamin B6 ist vorzugsweise in einer Menge von 0,1 mg bis 10 mg, insbesondere 0,5 mg bis 5 mg und besonders bevorzugt 0,8 mg bis 1,2 mg, in dem Präparat enthalten.

Weiterhin besonders bevorzugt enthält das Präparat Vitamin B1. Vitamin B1 ist auch unter dem Namen Thiamin bekannt. Es trägt ebenfalls zur Verbesserung des Biorhythmus' bei und hat schlafanstoßende Wirkung. Vitamin B1 ist vorzugsweise in einer Menge von 0,1 mg bis 10 mg, insbesondere 0,4 mg bis 1,2 mg und besonders bevorzugt 0,6 mg bis 1 mg, in dem Präparat enthalten.

Weiterhin besonders bevorzugt enthält das Präparat Folsäure. Folsäure wird auch als Vitamin B9 bezeichnet. Folsäure ist vorzugsweise in einer Menge von 0,025 mg bis 2,5 mg, insbesondere 0,1 mg bis 0,4 mg und besonders bevorzugt 0,15 mg bis 0,3 mg, in dem Präparat enthalten. Vorzugsweise ist die Folsäure als Glucosaminfolat enthalten.

Im Falle von Coenzym Q10 und Nicotinamid sind sowohl eine Konfektionierung in der ersten Phase wie auch in der zweiten Phase sehr gut geeignet. Ebenso ist es möglich, die Verbindungen in beliebigem Verhältnis auf die beiden Phasen aufzuteilen.

Coenzym Q10 ist vorzugsweise in einer Menge von 10 mg bis 100 mg, insbesondere 20 mg bis 60 mg und besonders bevorzugt von 30 mg bis 50 mg, in dem Präparat enthalten.

Nicotinamid ist das Amid der Nicotinsäure, die auch als Vitamin B3 bekannt ist. Nicotinamid ist vorzugsweise in einer Menge von 0,5 mg bis 5 mg, insbesondere 1 mg bis 4 mg und besonders bevorzugt zu 2,5 mg, in dem Präparat enthalten.

Coenzym Q10 und Nicotinamid sind wie auch Vitamin C, Zink, Kupfer und D-Biotin sind haut- und haarschützende Inhaltsstoffe, und sie sorgen somit für einen verbesserten Schutz von Haut und Haaren. Sie wirken der Austrocknungsgefahr entgegen, mildern die Wirkung verstärkter Sonneneinwirkung ab und beugen so der durch die verstärkte Sonneneinstrahlung und trockene Luft begünstigten beschleunigten Hautalterung vor.

Curcuma-Extrakt, Ingwer-Extrakt, Cranberry-Extrakt und Selen wirken ebenso wie Folsäure, Vitamin B6, Vitamin B12, Vitamin C und Zink antientzündlich und das Immunsystem stärkend. Diese Verbindungen sorgen somit dafür, dass das erfindungsgemäße Präparat die Abwehrkraft gegen für den Körper ungewohnte Keime stärkt und den Körper so schützt.

In einer weiteren bevorzugten Ausführungsform enthält die zweite Phase neben Magnesium und Tryptophan weiterhin Melissenextrakt und/oder Passionsblumenextrakt. Wie auch Tryptophan und Magnesium wirken beide Verbindungen beruhigend und schlaffördernd. Auch tragen diese Verbindungen zur Regeneration des Nervensystems bei. Sowohl Melissenextrakt als auch Passionsblumenextrakt sind daher, sofern vorhanden, vorteilhafterweise in der zweiten Phase enthalten.

Melissen (lat. Name: Melissa officinalis)-Extrakt enthält als Wirkstoff insbesondere Rosmarinsäure. Melissen-Extrakt kommt vorzugsweise in einer Menge von 2 mg. bis 100 mg, insbesondere 10 mg bis 50 mg und besonders bevorzugt 20 bis 30 mg zum Einsatz. Bevorzugte Rosmarinsäuren-Gehalte des Präparats liegen bei 0,1 mg bis 2 mg, insbesondere 0,5 mg bis 1 mg und besonders bevorzugt 0,7 mg bis 0,8 mg.

Passionsblumen (lat. Name: Passiflora)-Extrakt enthält als Wirkstoff insbesondere die Gruppe der Flavonoide. Passionsblumen-Extrakt kommt vorzugsweise in einer Menge von 2 mg. bis 100 mg, insbesondere 5 mg bis 50 mg und besonders bevorzugt 10 bis 30 mg zum Einsatz. Bevorzugte Flavonoid-Gehalte des Präparats liegen bei 0,1 mg bis 2 mg, insbesondere 0,6 mg bis 1 mg und besonders bevorzugt 0,7 mg bis 0,9 mg.

Laktobakterien und Bifidobakterien machen den Darm widerstandsfähiger. Bei diesen beiden Substanzen ist es besonders bevorzugt, wenn sie sowohl in der ersten wie auch in der zweiten Phase enthalten sind. Auf diese Weise ist es möglich, auch eine große Menge an Bakterien in dem 2-Phasen-Präparat zur Verfügung zu stellen. Eine bevorzugte Menge an Bakterien im Präparat liegt bei 1 Milliarde bis 20 Milliarden Bakterien (entsprechend 20 mg bis 400 mg Bakterien), insbesondere 5 Milliarden bis 10 Milliarden Bakterien (entsprechend 100 mg bis 200 mg), die jeweils vorzugsweise als Mischung aus Lakto- und Bifidobakterien vorliegen. Die Menge kann beliebig auf die beiden Phasen aufgeteilt werden. Vorzugsweise erfolgt eine gleichmäßige Aufteilung, so dass jede Phase vorzugsweise 0,5 Milliarden bis 10 Milliarden Bakterien, insbesondere 2,5 Milliarden bis 5 Milliarden Bakterien enthält.

Mariendistelextrakt weist eine leberschützende Funktion auf. Gemeinsam mit Selen, Zink, Kuper und Vitamin C, die vor oxidativem Stress schützen, stellt Mariendistelextrakt eine gesteigerte Entgiftungsfunktion zur Verfügung und kann somit die oftmals ungesunde Lebensweise auf Reisen, z.B. bedingt durch erhöhten Alkoholkonsum, zumindest teilweise kompensieren.

In einer besonders bevorzugten Zusammensetzung enthält das 2-Phasen-Präparat in der ersten Phase D-Biotin und Vitamin C, in der zweiten Phase Magnesium und Tryptophan und in mindestens einer der beiden Phasen weiterhin Cyanocobalamin, Coenzym Q10, Laktobakterien und Bifidobakterien, Zink, Vitamin D, Kupfer, Selen, Ingwer-Extrakt, Curcuma-Extrakt, Cranberry-Extrakt, Mariendistelextrakt sowie Melissenextrakt. Vorzugsweise sind dabei Coenzym Q10, Zink, Vitamin D, Selen, Ingwer-Extrakt, Curcuma-Extrakt und Cranberry-Extrakt in der ersten Phase, Kupfer, Mariendistelextrakt sowie Melissenextrakt in der zweiten Phase und die Laktobakterien und Bifidobakterien in beiden Phasen enthalten.

In einer anderen besonders bevorzugten Zusammensetzung enthält das 2-Phasen-Präparat in der ersten Phase D-Biotin und Vitamin C, in der zweiten Phase Magnesium und Tryptophan und in mindestens einer der beiden Phasen weiterhin Cyanocobalamin, Coenzym Q10, Laktobakterien und Bifidobakterien, Zink, Vitamin D, Kupfer, Selen, Ingwer-Extrakt, Curcuma-Extrakt, Cranberry-Extrakt, Mariendistelextrakt sowie Melissenextrakt und Passionsblumenextrakt. Vorzugsweise sind dabei Coenzym Q10, Zink, Vitamin D, Selen, Ingwer-Extrakt, Curcuma-Extrakt und Cranberry-Extrakt in der ersten Phase, Kupfer, Mariendistelextrakt sowie Melissenextrakt und Passionsblumenextrakt in der zweiten Phase und die Laktobakterien und Bifidobakterien in beiden Phasen enthalten.

In einer weiteren besonders bevorzugten Zusammensetzung enthält das 2-Phasen-Präparat in der ersten Phase D-Biotin und Vitamin C, in der zweiten Phase Magnesium und Tryptophan und in mindestens einer der beiden Phasen weiterhin Cyanocobalamin, Coenzym Q10, Laktobakterien und Bifidobakterien, Zink, Vitamin D, Kupfer, Selen, Ingwer-Extrakt, Curcuma-Extrakt, Cranberry-Extrakt, Mariendistelextrakt sowie Melissenextrakt, Passionsblumenextrakt und L-Glutamin. Vorzugsweise sind dabei Coenzym Q10, Zink, Vitamin D, Selen, Ingwer-Extrakt, Curcuma-Extrakt und Cranberry-Extrakt in der ersten Phase, Kupfer, L-Glutamin, Mariendistelextrakt sowie Melissenextrakt und Passionsblumenextrakt in der zweiten Phase und die Laktobakterien und Bifidobakterien in beiden Phasen enthalten.

In noch einer besonders bevorzugten Zusammensetzung enthält das 2-Phasen-Präparat in der ersten Phase D-Biotin und Vitamin C, in der zweiten Phase Magnesium und Tryptophan und in mindestens einer der beiden Phasen weiterhin Cyanocobalamin, Coenzym Q10, Laktobakterien und Bifidobakterien, Zink, Vitamin D, Kupfer, Selen, Ingwer-Extrakt, Curcuma-Extrakt, Cranberry-Extrakt, Mariendistelextrakt sowie Melissenextrakt, Passionsblumenextrakt, L-Glutamin und Nicotinamid. Vorzugsweise sind dabei Coenzym Q10, Zink, Vitamin D, Selen, Ingwer-Extrakt, Curcuma-Extrakt und Cranberry-Extrakt in der ersten Phase, Kupfer, L-Glutamin, Nicotinamid, Mariendistelextrakt sowie Melissenextrakt und Passionsblumenextrakt in der zweiten Phase und die Laktobakterien und Bifidobakterien in beiden Phasen enthalten.

In noch einer weiteren besonders bevorzugten Zusammensetzung enthält das 2-Phasen-Präparat in der ersten Phase D-Biotin und Vitamin C, in der zweiten Phase Magnesium und Tryptophan und in mindestens einer der beiden Phasen weiterhin Cyanocobalamin, Coenzym Q10, Laktobakterien und Bifidobakterien, Zink, Vitamin D, Kupfer, Selen, Ingwer-Extrakt, Curcuma-Extrakt, Cranberry-Extrakt, Mariendistelextrakt sowie Melissenextrakt, Passionsblumenextrakt, L-Glutamin, Nicotinamid und Vitamin B1. Vorzugsweise sind dabei Coenzym Q10, Zink, Vitamin D, Selen, Ingwer-Extrakt, Curcuma-Extrakt und Cranberry-Extrakt in der ersten Phase, Kupfer, L-Glutamin, Nicotinamid, Vitamin B1, Mariendistelextrakt sowie Melissenextrakt und Passionsblumenextrakt in der zweiten Phase und die Laktobakterien und Bifidobakterien in beiden Phasen enthalten.

In noch einer anderen besonders bevorzugten Zusammensetzung enthält das 2-Phasen-Präparat in der ersten Phase D-Biotin und Vitamin C, in der zweiten Phase Magnesium und Tryptophan und in mindestens einer der beiden Phasen weiterhin Cyanocobalamin, Coenzym Q10, Laktobakterien und Bifidobakterien, Zink, Vitamin D, Kupfer, Selen, Ingwer-Extrakt, Curcuma-Extrakt, Cranberry-Extrakt, Mariendistelextrakt sowie Melissenextrakt, Passionsblumenextrakt, L-Glutamin, Nicotinamid, Vitamin B1 und Folsäure. Vorzugsweise sind dabei Coenzym Q10, Zink, Vitamin D, Selen, Ingwer-Extrakt, Curcuma-Extrakt und Cranberry-Extrakt in der ersten Phase, Kupfer, L-Glutamin, Nicotinamid, Vitamin B1, Folsäure, Mariendistelextrakt sowie Melissenextrakt und Passionsblumenextrakt in der zweiten Phase und die Laktobakterien und Bifidobakterien in beiden Phasen enthalten.

In noch einer alternativen besonders bevorzugten Zusammensetzung enthält das 2-Phasen-Präparat in der ersten Phase D-Biotin und Vitamin C, in der zweiten Phase Magnesium und Tryptophan und in mindestens einer der beiden Phasen weiterhin Cyanocobalamin, Coenzym Q10, Laktobakterien und Bifidobakterien, Zink, Vitamin D, Kupfer, Selen, Ingwer-Extrakt, Curcuma-Extrakt, Cranberry-Extrakt, Mariendistelextrakt sowie Melissenextrakt, Passionsblumenextrakt, L-Glutamin, Nicotinamid, Vitamin B1, Folsäure und Vitamin B6. Vorzugsweise sind dabei Coenzym Q10, Zink, Vitamin D, Selen, Ingwer-Extrakt, Curcuma-Extrakt und Cranberry-Extrakt in der ersten Phase, Kupfer, L-Glutamin, Nicotinamid, Vitamin B1, Vitamin B6, Folsäure, Mariendistelextrakt sowie Melissenextrakt und Passionsblumenextrakt in der zweiten Phase und die Laktobakterien und Bifidobakterien in beiden Phasen enthalten.

Neben den genannten Wirkstoffen können noch weitere Wirkstoffe wie weitere Vitamine, Mineralien oder andere Naturstoffe enthalten sein. Besonders bevorzugt sind außer den vorstehend genannten Wirkstoffen jedoch keine weiteren Wirkstoffe in dem Präparat enthalten.

Bevorzugte Darreichungsformen des erfindungsgemäßen 2-Phasen-Präparates sind Kapseln, insbesondere Cellulose- sowie Weich- oder Hartgelatinekapseln, Pulver, Tabletten, insbesondere Brausetabletten, Lutschtabletten, Kautabletten oder Dragees, Lösungen, Säfte oder Sirupe.

Besonders bevorzugt sind die beiden Phasen unter Verwendung von Hilfsstoffen jeweils als Kapsel konfektioniert, wobei Kapseln aus nicht-tierischen Rohstoffen, insbesondere Cellulose, bevorzugt werden, da diese auch mit einer vegetarischen oder veganen Lebensweise vereinbar sind. Weiterhin sollten die Kapseln eine gewisse Säurebeständigkeit aufweisen und nicht bereits in einem frühen Stadium im Magen die Inhaltsstoffe freisetzen, sondern erst zu einem späteren Zeitpunkt im Magen-DarmTrakt, was eine bessere Resorption der Wirkstoffe mit sich bringt.

Das erfindungsgemäße Präparat kann neben den genannten Wirkstoffen auch sogenannte Hilfsstoffe umfassen, die der Fachmann je nach Anwendung frei wählen kann.

Dabei sind als Träger bzw. Füllstoffe Glycerin, Mono-, Di- und Triglyceride, Magnesiumstearate, Stärke, Cellulose, Stärkederivate sowie Cellulosederivate und weiterhin Pflanzenöle wie Rapsöl oder Kokosöl besonders geeignet.

Bevorzugte Bindemittel sind Polyvinylpyrrolidone sowie Polyethylenglykole oder Gelatine.

Als Trenn- bzw. Gleitmittel kommen vorteilhafter Weise Siliziumdioxid, Magnesiumstearat, Lecithin, Cellulose und/oder Carboxymethylstärke zum Einsatz.

Für einen besonders angenehmen Geschmack kann das Präparat zusätzlich Süßungsmittel und/oder Aromen umfassen, wobei Maltodextrin, Fructose, Glucose und Sorbit vorzugsweise zum Einsatz kommen.

Für einen besonders ansprechenden optischen Eindruck können dem Präparat darüber hinaus Farbstoffe zugesetzt sein, vorzugsweise Titandioxid (E171) sowie rotes Eisenoxid (E172).

Vorzugsweise sind die verwendeten Hilfsstoffe vegan. Damit wird eine breite Akzeptanz des erfindungsgemäßen Nahrungsergänzungsmittels erreicht. Besonders bevorzugt kommt dabei als Trägermaterial Cellulose zum Einsatz.

Weiterhin kann dem Nahrungsergänzungsmittel ein Farbstoff zugesetzt sein. Zum einen wird durch ein Einfärben der optische Eindruck ansprechender, zum anderen ist auf diese Weise eine einfache Unterscheidung der beiden Phasen möglich. So kann zum Beispiel für die erste Phase eine weiße Kapsel verwendet werden, für die zweite Phase eine rote oder grüne Kapsel.

Das erfindungsgemäße Präparat ist, insbesondere wenn es in Form von Kapseln, Tabletten oder Dragees konfektioniert ist, sehr einfach einzunehmen. Eine Einnahme mit Flüssigkeit ist bevorzugt. Notfalls kann das Präparat jedoch auch ohne Flüssigkeit eingenommen werden.

Erfindungsgemäß wird das vorstehend beschriebene Nahrungsergänzungsmittel zur Vorbeugung vor und Linderung von reisebedingten Beschwerden verwendet. Die reisebedingten Beschwerden sind vorstehend ausführlich beschrieben.

Dabei wird die erste Phase morgens und die zweite Phase abends eingenommen. Wenn die beiden Phasen beispielsweise in Form von Kapseln konfektioniert sind, kann das Präparat in Form einer Morgenkapsel und einer Abendkapsel eingenommen werden. Die Morgenkapsel wird nach dem Aufstehen, vorzugsweise nach dem Frühstück, eingenommen. Die Abendkapsel wird abends vor dem Schlafengehen, vorzugsweise eine Stunde vor dem Schlafengehen, eingenommen.

Besonders bevorzugt wird mit der Einnahme des Präparates bereits 3 Tage vor Beginn der Reise begonnen. Die Einnahmezeitpunkte richten sich dabei nach der Zeitzone des Ausgangsortes. Bei Erreichen des Reiseziels wird für die Einnahmezeitpunkte auf die Zeitzone des Zielortes abstellt. Weiterhin wird das Präparat auch noch 3 Tage nach Beendigung der Reise eingenommen. Auch hier wird für den Zeitpunkt der Einnahme wieder auf die Zeitzone des Heimatortes abgestellt.

Die vorliegende Anmeldung betrifft schließlich auch die Verwendung des vorstehend beschriebenen 2- Phasen-Präparats zur Herstellung eines zirkadianen Nahrungsergänzungsmittels zur Vorbeugung vor und Linderung von reisebedingten Beschwerden.

Beispielrezeptur:
Ein erfindungsgemäßes 2-Phasen-Präparat wird in Form von zwei Kapseln hergestellt. Die beiden Kapseln enthalten folgende Wirkstoffzusammensetzung:

### Morgenkapsel

| **Zusammensetzung** | **pro Tagesportion** |
|---|---|
| Probiotische Bakterienmischung | 100,00 mg entspr. 5 Milliarden Bakterien |
| Vitamin C | 74,20 mg |
| Curcuma longa-Extrakt | 70,00 mg |
| davon Curcumin | 49,00 mg |
| Ingwerwurzel-Extrakt (Zingiber officinale) | 70,00 mg |
| davon Gingerole | 3,50 mg |
| Cranberry-Extrakt (Vaccinium macrocarpon) | 50,00 mg |
| davon Proanthocyanidine | 12,50 mg |
| Coenzym Q10 | 40,00 mg |
| Zink | 10,00 mg |
| Selen | 0,0675 mg |
| D-Biotin (Vitamin H) | 0,0500 mg |
| Cholecalciferol (Vitamin D3) | 0,0200 mg entspr. 800 E |

### Abendkapsel

| **Zusammensetzung** | **pro Tagesportion** |
|---|---|
| Mariendistelextrakt (Silybum marianum) | 100,00 mg |
| davon Silymarin | 80,00 mg |
| Probiotische Bakterienmischung | 100,00 mg entspr. 5 Milliarden Bakterien |
| L-Tryptophan | 98,50 mg |
| Magnesium | 60,30 mg |
| L-Glutamin | 25,00 mg |
| Melissenextrakt (Melissa officinalis) | 25,00 mg |
| davon Rosmarinsäure | 0,75 mg |
| Passionsblumenextrakt (Passiflora incarnata) | 20,00 mg |
| davon Flavonoide | 0,80 mg |
| Nicotinamid (Vitamin B3) | 2,5000 mg |
| Pyridoxin (Vitamin B6) | 0,9900 mg |
| Thiamin (Vitamin B1) | 0,8100 mg |
| Folsäure | 0,2160 mg |
| Kupfer | 0,50 mg |
| Cyanocobalamin (Vitamin B12) | 0,0055 mg |

Vitamin C ist in der Beispielrezeptur als Calciumascorbat, Magnesium als Magnesiumcitrat, Zink als Zinkcitrat, Selen als Natriumselenit, Folsäure als Glucosaminfolat und Kupfer als Kupfergluconat enthalten. Die Mengenangaben in der vorstehenden Tabelle beziehen sich jeweils auf die reinen Wirkstoffe (also Vitamin C, Magnesium, Zink, Selen, Folsäure und Kupfer) ohne Berücksichtigung der Gegenionen.

Als Füllstoff sowie für die Kapselhülle wird Cellulose verwendet. Ferner wird der Morgenkapsel als Farbstoff Titandioxid (E171), der Abendkapsel rotes Eisenoxid (E172) zugesetzt.

Die Einnahme der Morgenkapsel erfolgt nach dem Frühstück, die der Abendkapsel eine Stunde vor dem Schlafengehen. Die Einnahme der beiden Kapseln wird drei Tage vor Beginn der Reise begonnen und noch drei Tage nach Ende der Reise fortgesetzt.

Das Präparat wurde von mehreren Probanden getestet. Die Probanden unternahmen Reisen mit dem Flugzeug, wobei sie in ein Gebiet reisten, das in einer Zeitzone mit einem Zeitunterschied von mindestens vier Stunden zum Ausgangsort aufwies. Dabei wurde übereinstimmend berichtet, dass die mit einem Ortswechsel einhergehenden Beschwerden gemildert wurden. Konkret wurde angegeben, dass man sich mit dem Präparat frischer fühle, besser schlafen könne und Alkoholkonsum besser toleriere als bei bisherigen Reisen, bei denen das Präparat nicht genommen wurde. Ferner berichteten die Probanden, dass sie ein besseres Hautbild bei sich beobachtet hätten als dies bei zuvor unternommenen Flugreisen ohne Einnahme des Präparats der Fall gewesen sei. Erkältungssymptome oder Magen-Darm-Beschwerden wurden von keinem der Probanden angegeben, was für den immunitätsverbessernden Effekt spricht, auch im Darmbereich. Eine Probandin wies darauf hin, dass sie sonst auf Reisen immer "mit dem Darm zu tun" habe, was unter dem Präparat nicht der Fall gewesen sei.

## Patentansprüche

1. Zirkadianes Nahrungsergänzungsmittel zur Verwendung bei der Vorbeugung vor und Linderung von reisebedingten Beschwerden bestehend aus einem 2-Phasen-Präparat, wobei
die erste Phase D-Biotin und Vitamin C enthält,
die zweite Phase Magnesium und Tryptophan enthält und
mindestens eine der beiden Phasen weiterhin Folgendes enthält:
- Cyanocobalamin
- Coenzym Q10
- Laktobakterien und Bifidobakterien
- Zink
- Vitamin D
- Kupfer
- Selen
- Ingwer-Extrakt
- Curcuma-Extrakt
- Cranberry-Extrakt
- Mariendistelextrakt
- sowie optional eine oder mehrere der folgenden Substanzen:
- L-Glutamin
- Nicotinamid
- Vitamin B6
- Vitamin B1
- Folsäure
- Melissenextrakt
- Passionsblumenextrakt,
**dadurch gekennzeichnet, dass** die erste Phase morgens und die zweite Phase abends eingenommen wird.

2. Zirkadianes Nahrungsergänzungsmittel nach Anspruch 1, **dadurch gekennzeichnet, dass** die zweite Phase
- Mariendistelextrakt
- L-Glutamin
- Nicotinamid
- Vitamin B6
- Vitamin B1
- Cyanocobalamin
- Folsäure und
- Kupfer
enthält.

3. Zirkadianes Nahrungsergänzungsmittel nach Anspruch 1 oder 2, **dadurch gekennzeichnet, dass** die erste Phase
- Coenzym Q10
- Curcuma-Extrakt
- Ingwer-Extrakt
- Cranberry-Extrakt
- Selen
enthält.

4. Zirkadianes Nahrungsergänzungsmittel nach einem der Ansprüche 1 bis 3, **dadurch gekennzeichnet, dass** die beiden Phasen unter Verwendung von Hilfsstoffen jeweils als Kapsel konfektioniert sind.

5. Zirkadianes Nahrungsergänzungsmittel nach Anspruch 4, **dadurch gekennzeichnet, dass** die verwendeten Hilfsstoffe vegan sind.

## Claims

1. Circadian dietary supplement for use in the prevention and relief of travel-related ailments, consisting of a 2-phase-preparation, wherein
the first phase contains D-biotin and vitamin C,
the second phase contains magnesium and tryptophan and
at least one of the two phases further contains the following:
- cyano cobalamine
- coenzyme Q10
- lactic acid bacteria and bifido bacteria
- zinc
- vitamin D
- copper
- selenium
- ginger extract
- curcuma extract
- cranberry extract
- milk thistle extract
- and optionally one or more of the following substances:
- L-glutamine
- nicotine amide
- vitamin B6
- vitamin B1
- folic acid
- balm extract
- passion flower extract,
**characterized in that** the first phase is taken in the morning and the second phase is taken in the evening.

2. Circadian dietary supplement according to claim 1, **characterized in that** the second phase contains
- milk thistle extract
- L-glutamine
- nicotine amide
- vitamin B6
- vitamin B1
- cyano cobalamine
- folic acid and
- copper.

3. Circadian dietary supplement according to claim 1 or 2, **characterized in that** the first phase contains
- coenzyme Q10
- curcuma extract
- ginger extract
- cranberry extract
- selenium.

4. Circadian dietary supplement according to one of claims 1 to 3, **characterized in that** the two phases are each manufactured as a capsule using excipients.

5. Circadian dietary supplement according to claim 4, **characterized in that** the used excipients are vegan.

## Revendications

1. Complément alimentaire circadien destiné à être utilisé dans la prévention et le soulagement des troubles liés au voyage consistant en une préparation à deux phases,
la première phase contient de la D-biotine et de la vitamine C,
la deuxième phase contient du magnésium et du tryptophane, et
au moins une des deux phases contient en outre ce qui suit :
- cyanocobalamine
- coenzyme Q10
- lactobactéries et bifidobactéries
- zinc
- vitamine D
- cuivre
- sélénium
- extrait de gingembre
- extrait de curcuma
- extrait de canneberge
- extrait de chardon-marie
- ainsi que, en option, une ou plusieurs des substances suivantes :
- L-glutamine
- nicotinamide
- vitamine B6
- vitamine B1
- acide folique
- extrait de mélisse
- extrait de passiflore,
**caractérisé en ce que** la première phase est prise le matin et la deuxième phase le soir.

2. Complément alimentaire circadien selon la revendication 1, **caractérisé en ce que** la deuxième phase contient
- extrait de chardon-marie
- L-glutamine
- nicotinamide
- vitamine B6
- vitamine B1
- cyanocobalamine
- acide folique et
- cuivre.

3. Complément alimentaire circadien selon la revendication 1 ou 2, **caractérisé en ce que** la première phase contient
- coenzyme Q10
- extrait de curcuma
- extrait de gingembre
- extrait de canneberge
- sélénium.

4. Complément alimentaire circadien selon l'une des revendications 1 à 3, **caractérisé en ce que** les deux phases sont confectionnées chacune sous forme de capsule en utilisant des excipients.

5. Complément alimentaire circadien selon la revendication 4, **caractérisé en ce que** les excipients utilisés sont végétaliens.
